# FASCICULE DE BREVET EUROPEEN

(11) **EP 1 204 744 B1**
(45) Date de publication et mention de la délivrance du brevet: **08.03.2006**
(21) Numéro de dépôt: 00922701.8
(22) Date de dépôt: 20.04.2000
(51) Int. Cl.: C12N 15/12, C07K 14/47, C07K 16/18, A61K 38/17, A61K 48/00

(54) **POLYPEPTIDE ISOLE DU STRATUM CORNEUM ET SON UTILISATION**
POLYPEPTID AUS STRATUM CORNEUM UND SEINE VERWENDUNG
ISOLATED PEPTIDE OF THE HORNY LAYER AND USE THEREOF

(30) Priorité: 23.07.1999 FR 9909615
(43) Date de publication de la demande: 15.05.2002
(73) Titulaire: L'OREAL, 75008 Paris (FR)
(72) Inventeur: MEHUL, Bruno, F-92800 Villejuif (FR); BERNARD, Dominique, F-75015 Paris (FR); SIMONETTI, Lucie, F-75011 Paris (FR)
(74) Mandataire: Allab, Myriam
(86) Numéro de dépôt international: PCT/FR2000/001048
(87) Numéro de publication internationale: WO 2001/007604

(56) Documents cités:
- EP-A- 0 731 166
- FR-A- 2 761 363
- US-A- 5 763 220
- US-A- 6 046 315
- DATABASE EMBL/HINXTON, U.K. [en ligne] "ni01f09.y5 NCI_CGAP_Br2 Homo sapiens cDNA clone IMAGE:966761 5' similar to SW:CALM_PLAFA P24044 CALMODULIN" XP002137649
- WILKINSON, M. M. ET AL.: "Expression pattern of two related cystic fibrosis-associated calcium-binding proteins in normal and abnormal tissues." J. CELL SCI., vol. 91, Pt2, octobre 1988 (1988-10), pages 221-230, XP000907159
- SCHAFER B W ET AL: "The S100 family of EF-hand calcium-binding proteins: functions and pathology" TIBS TRENDS IN BIOCHEMICAL SCIENCES,EN,ELSEVIER PUBLICATION, CAMBRIDGE, vol. 21, no. 4, 1 avril 1996 (1996-04-01), pages 134-140, XP004050923 ISSN: 0968-0004
- POLANS A S ET AL: "A PHOTORECEPTOR CALCIUM BINDING PROTEIN IS RECOGNIZED BY AUTOANTIBODIES OBTAINED FROM PATIENTS WITH CANCER-ASSOCIATED RETINOPATHY" JOURNAL OF CELL BIOLOGY,US,NEW YORK, US, vol. 112, no. 5, 1 mars 1991 (1991-03-01), pages 981-989, XP000572591 ISSN: 0021-9525
- NAIK U P ET AL: "IDENTIFICATION OF A NOVEL CALCIUM-BINDING PROTEIN THAT INTERACTS WITH THE INTEGRIN ALPHAIIB CYTOPLASMIC DOMAIN" JOURNAL OF BIOLOGICAL CHEMISTRY,US,AMERICAN SOCIETY OF BIOLOGICAL CHEMISTS, BALTIMORE, MD, vol. 272, no. 8, 21 février 1997 (1997-02-21), pages 4651-4654, XP002056117 ISSN: 0021-9258
- PETERSON, L.L. ET AL.: "Purification of Human Epidermal Calmodulin and Its Activation of Epidermal Transglutaminase" CLINICAL RESEARCH, vol. 30, no. 1, 1982, page A159 XP000946386
- PUSZKIN, E. G. ET AL.: "Catalytic Properties of a Calmodulin-regulated Transglutaminase from Human Platelet and Chicken Gizzard" J. BIOL. CHEM., vol. 260, no. 29, 15 décembre 1985 (1985-12-15), pages 16012-16020, XP002148604
- LUNDSTRÖM AND EGELRUD: "Stratum Corneum Chymotryptic Enzyme: A Proteinase Which May Be Generally Present in the Stratum Corneum and With a Possible Involvement in Desquamation" ACTA DERM VENEREOL, vol. 71, 1991, pages 471-474, XP000749556 STOCKHOLM

### LISTE DE SEQUENCES

<110> L'OREAL
<120> Polypeptide isolé du stratum corneum et son utilisation
<130> OA99236
<140>
   <141>
<160> 2
<170> PatentIn Ver. 2.1
<210> 1
   <211> 146
   <212> PRT
   <213> Homo sapiens
<400> 1
   Met Ala Gly Glu Leu Thr Pro Glu Glu Glu Ala Gln Tyr Lys Lys Ala
   1 5 10 15
   Phe Ser Ala Val Asp Thr Asp Gly Asn Gly Thr Ile Asn Ala Gln Glu
   20 25 30
   Leu Gly Ala Ala Leu Lys Ala Thr Gly Lys Asn Leu Ser Glu Ala Gln
   35 40 45
   Leu Arg Lys Leu Ile Ser Glu Val Asp Ser Asp Gly Asp Gly Glu Ile
   50 55 60
   Ser Phe Gln Glu Phe Leu Thr Ala Ala Arg Lys Ala Arg Ala Gly Leu
   65 70 75 80
   Glu Asp Leu Gln Val Ala Phe Arg Ala Phe Asp Gln Asp Gly Asp Gly
   85 90 95
   His Ile Thr Val Asp Glu Leu Arg Arg Ala Met Ala Gly Leu Gly Gln
   100 105 110
   Pro Leu Pro Gln Glu Glu Leu Asp Ala Met Ile Arg Glu Ala Asp Val
   115 120 125
   Asp Gln Asp Gly Arg Val Asn Tyr Glu Glu Phe Ala Arg Met Leu Ala
   130 135 140
   Gln Glu
   145
<210> 2
   <211> 858
   <212> ADN
   <213> Homo sapiens
<400> 2
   aattcccgga tccctgcggc tgcctgcact ctggaccacg agctctgaga gcagcaggtt 60
   gagggccggt gggcagcagc tcggaggctc cgcgaggtgc aggagacgca ggcatggccg 120
   gtgagctgac tcctgaggag gaggcccagt acaaaaaggc tttctccgcg gttgacacgg 180
   atggaaacgg caccatcaat gcccaggagc tgggcgcggc gctgaaggcc acgggcaaga 240
   acctctcgga ggcccagcta aggaaactca tctccgaggt tgacagcgac ggcgacggcg 300
   aaatcagctt ccaggagttc ctgacggcgg caaggaaggc cagggccggc ctggaggacc 360
   tgcaggtcgc cttccgcgcc ttcgaccagg atggcgacgg ccacatcacc gtggacgagc 420
   tcaggcgggc catggcgggg ctggggcagc cgctgccgca ggaggagctg gacgccatga 480
   tccgcgaggc cgacgtggac caggacgggc gggtgaacta cgaggagttc gcgaggatgc 540
   tcgcccagga gtgaggctcc ccgcctgtgt ccccctggct gcgctctgag ccttcagggc 600
   caccgcccgc tgctgctttt gtgctgggac tctccgggga aacctggtcg gtggatggga 660
   aactgcctcc ccctgggagg aaggctttgc gctccggggc ctggatgcgg cgccctcggg 720
   ccgcctgcga gcccctctct gccttcagac cttgggcaga aggaggcctc cttgggcctg 780
   gtcccccttt gccctgcagt ggaatgaggg ccccttaacc ccgcattgat ctaaataaag 840
   gactgccgag ttccaaaa                                              858

## Description

L'invention a pour objet un polypeptide isolé, de la famille des protéines fixant le calcium, un mélange de polypeptides issus de la protéolyse du polypeptide isolé, des compositions les contenant, les utilisations dudit polypeptide et un procédé de traitement cosmétique destiné à traiter les peaux sèches, l'hyperkératose, la parakératose, le psoriasis, l'ichtyose, les néoplasies. L'invention a également pour objet une séquence d'acide désoxyribonucléique codant pour ledit polypeptide et les utilisations de ladite séquence d'acide désoxyribonucléique.

Le rôle clef des flux de calcium dans les fonctions cellulaires a été largement décrit depuis plus de 20 ans. Ainsi le calcium est un messager intracellulaire de première importance. De nombreuses hormones et messagers extracellulaires exercent leur effet par une augmentation du taux intracellulaire de calcium. Il a été rapidement montré que la plupart des effets du calcium étaient déterminés par une large famille relativement homogène de protéines liant le calcium. Parmi cette famille les Calmodulines sont les plus ubiquitaires. Le rôle de la Calmoduline est de reconnaître les changements dans la concentration en ions calciques cytosoliques et de transmettre l'information aux protéines intracellulaires.
La transmission du signal est effectuée lorsque la Calmoduline lie le calcium lors d'une augmentation du taux cellulaire de celui-ci. Cela entraîne des changements de conformation de la protéine ce qui augmente de manière considérable son affinité pour la protéine cible.

En terme de configuration, la Calmoduline libre de calcium contient deux domaines globulaires connectés par un bras flexible. Chaque domaine contient deux motifs « Hélice-Boucie-Hélice » bien définis connus sous le nom de « EF-Hands » et qui sont responsables de la liaison avec le calcium. La liaison avec le calcium induit un changement conformationnel très important Ce changement conformationnel se traduit par une exposition de la partie hydrophobe globulaire de la protéine qui permet à la Calmoduline de reconnaître et de se lier à certaines protéines avec une haute affinité. La liaison de la Calmoduline avec sa protéine cible conduit alors à la création d'un complexe actif.

La Calmoduline est connue pour interagir avec de nombreuses protéines ou enzymes ainsi qu'avec de nombreuses drogues hydrophobes ou encore avec des peptides d'origine naturelle ou synthétique.

A cet égard on peut citer
- certaines protein-kinases impliquées dans l'homéostasie cellulaire et dans certaines fonctions cellulaires critiques telles que le métabolisme, la motilité, la transcription des gènes et la division cellulaire, la prolifération ;
- la calcineurin qui est une phosphatase ayant un rôle dans la régulation des enzymes et protéines impliquées dans la transduction du signal calcique;
- la phosphodiestérase à nucléotides cycliques, la guanydylcyclase et l'adénylylcyclase régulant ainsi de manière positive et/ou négative les taux des seconds messagers AMPcyclique et GMPcyclique dans le mécanisme d'action de certaines hormones ;
- les trois isoformes de la NO-synthase intervenant dans la production de NO et ainsi dans la relaxation vasculaire, l'action cytotoxique des macrophages, le relargage de neurotransmetteurs, de neuropeptides et/ou d'hormones ;
- certaines protéines du cytosquelette vitales dans le contrôle de la forme des cellules, de leur rigidité et de leur adhésion comme par exemple la myosine ;
- la caldesmon, les spectrines, l'adducine, protéines liant l'actine ;
- la desmocalmine, une protéine du desmosome qui interagit avec les kératines ;
- les plasma-membrane Ca²⁺ -ATPases intervenant dans la maintenance de la concentration intracellulaire en ion ;
- l'ornithine décarboxylase, certaines phospholipases A2 et certaines transglutaminases ont été citées comme étant calmodulin-dépendantes.

D'autre part, certaines études suggèrent que la Calmoduline a un rôle dans les phénomènes de réparation de l'acide désoxyribonucléique et qu'elle serait potentiellement impliquée dans le vieillissement cutané.

Une séquence partielle d'ARNm issu de tissu de sein cancéreux et utilisé comme EST a déjà été identifiée par Strausberg et al. (AI 791495). Par ailleurs, il est connu que la calmoduline humaine purifiée à partir d'épiderme active les transglutaminases (Clinical research, Vol.30, n°1, 1982, pA159).

On sait enfin que si la Calmoduline est une protéine bien décrite, elle est en fait le prototype d'une large famille de protéines dont tous les individus n'ont pas encore été décrits.

Ainsi, après de longs et laborieux travaux la demanderesse a mis en évidence, parmi les protéines de l'épiderme, isolé et purifié par des techniques biochimiques, un polypeptide exprimé dans les épithélia cornifiés. Ce polypeptide, autrement appelé par ailleurs dans le texte "CLSP" (pour protéine de la peau similaire de la Calmoduline : Calmodulin Like Skin Protein) est exprimé dans l'épiderme. On sait que dans les épithélia comifiés la très grande majorité des protéines exprimées est constituée par les kératines. Ainsi, ce n'est qu'en élaborant une méthode d'extraction particulière éliminant les kératines, que la demanderesse a pu isoler puis purifier la CLSP.

La demanderesse en a alors déterminé la séquence primaire en acides aminés.

L'invention a donc pour objet un polypeptide isolé et purifié, appartenant à la famille des protéines fixant le calcium (CaBP : calcium binding protein), caractérisé par le fait qu'il répond à la séquence d'acides aminés SEQ ID NO : 1 suivante : Le polypeptide de l'invention peut être d'origine naturelle ou synthétique. Par synthétique on entend ici tout polypeptide obtenu chimiquement ou par production dans un organisme après introduction dans cet organisme des éléments nécessaires à cette production.

Le polypeptide de l'invention peut être issu de toute origine possible à savoir soit animale, en particulier de mammifères et encore plus particulièrement humaine, soit végétale, soit de micro-organismes (virus, phages, bactéries entre autres) ou encore de champignons, sans préjuger du fait qu'il soit présent de manière naturelle ou non dans ledit organisme d'origine.

Préférentiellement, le polypeptide de l'invention est d'origine naturelle, purifié à partir de tissus de mammifères, particulièrement à partir de peau de mammifères.

Préférentiellement, le polypeptide de l'invention est purifié à partir de peau humaine et encore plus préférentiellement à partir d'épiderme humain.

On sait que dans un polypeptide, un ou plusieurs résidus d'acide aminé peuvent changer pour des résidus d'acide aminé ayant un index hydropathique similaire sans pour autant changer les propriétés biologiques du polypeptide. L'index hydropathique est un index attribué aux acides aminés en fonction de leur hydrophobicité et de leur charge (Kyte et al. (1982), J. Mol. Biol., 157 : 105).
Ainsi l'invention a également pour objet un polypeptide tel que décrit ci-dessus dans lequel un résidu d'acide aminé au moins a été changé pour un résidu d'acide aminé ayant un index hydropathique similaire.

On sait qu'en général les polypeptides matures que l'on trouve dans les cellules proviennent de la maturation de précurseurs qui contiennent dans leur séquence la séquence du polypeptide mature.

Ainsi, l'invention concerne également tout polypeptide, naturel ou synthétique, dont la séquence est en partie constituée par la séquence du polypeptide de l'invention.

Il est connu que les polypeptides peuvent subir des modifications post-traductionnelles comme la formation de liaisons disulfures, les clivages protéolytiques spécifiques, l'addition de glucides (glycosylation), la phosphorylation en particulier au niveau des sérines et/ou des thréonines et/ou des tyrosines, et/ou l'association à des lipides.
Le polypeptide de l'invention peut avoir subi une ou plusieurs modifications post-traductionnelles.

Ainsi l'invention concerne aussi le polypeptide de l'invention ayant subi ou non des modifications post-traductionnelles.

On sait classer les polypeptides en fonction de leur point isoélectrique.
Le point isoélectrique théorique du polypeptide de l'invention peut être déduit de son enchaînement en acides aminés. Le polypeptide de l'invention est un polypeptide théoriquement acide.
Ainsi, l'invention a pour objet un polypeptide dont le point isoélectrique théorique est compris entre 1 et 6, particulièrement entre 3 et 5. Le polypeptide de l'invention a un point isoélectrique théorique de 4,075.

On sait que la séquence primaire en acides aminés ainsi que les diverses modifications post-traductionnelles subies par un polypeptide font que ledit polypeptide peut être caractérisé par son poids moléculaire apparent exprimé en kilodaltons.
On entend par poids moléculaire apparent, le poids moléculaire obtenu pour le polypeptide par comparaison de la mobilité électrophorétique de celui-ci avec celles de protéines standards de poids moléculaires connus sur gel de polyacrylamide/sodium dodécylsulfate, ou encore par comparaison du volume d'élution du polypeptide avec celui de protéines standard de poids moléculaires connus en chromatographie d'exclusion (selon les techniques décrites dans "Protein Purification", J-C. Janson et L. Ryden, VCH Publisher Inc. N.Y., 1989).

La connaissance de l'enchaînement en acides aminés du polypeptide de l'invention permet d'en déterminer le poids moléculaire théorique.
L'invention concerne donc un polypeptide ayant un poids moléculaire théorique compris entre 13 et 17 kilodaltons (kD), particulièrement entre 14 et 16 kilodaltons (kD).

Très particulièrement le polypeptide de l'invention a un poids moléculaire théorique de 15,9 kilodaltons (kD).

Il est par ailleurs connu que la séquence primaire en acides aminés d'un polypeptide détermine des sites spécifiquement reconnus par des protéases qui une fois la reconnaissance de ces sites effective vont, avec ou sans fixation audit polypeptide, induire son clivage par protéolyse.

Ainsi, l'invention concerne également au moins un mélange de polypeptides issus de la protéolyse du polypeptide de l'invention.

On comprend donc que dans le texte et sans indication contraire, par polypeptide il faut entendre le polypeptide naturel ou synthétique de l'invention tel que décrit ci-dessus ou au moins l'un de ses fragments, qu'il soit obtenu par protéolyse ou de manière synthétique ou encore tout polypeptide naturel ou synthétique dont la séquence est totalement ou partiellement constituée par la séquence du polypeptide précédemment décrit.

L'analyse de la séquence primaire ne acides aminés du polypeptide de l'invention montre que celle-ci contient des sites particulier reconnus comme participant, dans les polypeptides fixant le calcium, à l'élaboration du ou des sites de fixation du calcium. La fixation du calcium par le polypeptide de l'invention est d'ailleurs confirmée par les résultats des essais présentés par ailleurs dans les exemples.

Le polypeptide de l'invention est un polypeptide qui fixe le calcium.

On a vu par ailleurs dans le texte l'importance de la présence dans l'organisme des protéines de la famille de la Calmoduline. On comprend alors l'importance de pouvoir fournir à l'organisme une protéine de la famille de la Calmoduline afin de modifier les effets de celle-ci. Les applications potentielles couvrent donc toutes les voies de transduction du signal calcio-dépendante.

Par exemple en cosmétique la protéine de l'invention peut être utilisée afin de réguler les dysfonctionnements de la prolifération ou de la différenciation épidermique, normales ou pathologiques (peaux sèches, hyperkératose, parakératose, psoriasis, ichtyose, néoptasie...), dans le traitement du vieillissement, particulièrement du vieillissement cutané, dans le traitement des dommages cutanés liés à l'exposition aux rayonnements ultra-violet.

Ainsi, un autre objet de l'invention est donc de fournir des compositions comprenant, dans un milieu physiologiquement acceptable, au moins un polypeptide tel que décrit précédemment.

Un milieu physiologiquement acceptable est selon l'invention un milieu cosmétiquement et/ou pharmaceutiquement acceptable, compatible avec la peau, les muqueuses, les ongles, les cheveux.
Les compositions de l'invention peuvent être des compositions cosmétiques ou pharmaceutiques.

De préférence, les compositions de l'invention sont appliquées sur la peau ou les muqueuses.

Le polypeptide de l'invention peut être utilisé comme agent régulant les dysfonctionnements de la prolifération ou de la différenciation épidermique, normales ou pathologiques particulièrement dans le traitement des peaux sèches, de l'hyperkératose, de la parakératose, du psoriasis, des ichtyoses, des néoplasies. Le polypeptide de l'invention peut ainsi être introduit dans une composition destinée au soin et/ou au maquillage de la peau, des muqueuses et/ou des fibres kératiniques.
La composition de l'invention est aussi destinée à lutter contre les signes du vieillissement cutané et/ou à lutter contre les effets du rayonnement ultra-violet, particulièrement de type A ou B.

Un autre objet de l'invention est donc une composition destinée à traiter les dysfonctionnements de la prolifération ou de la différenciation épidermique, les peaux sèches, l'hyperkératose, la parakératose, le psoriasis, les ichtyoses et/ou les néoplasies.
Elle concerne aussi une composition comprenant au moins un polypeptide de l'invention pour lutter contre les signes du vieillissement cutané et/ou lutter contre les effets du rayonnement ultra-violet, particulièrement de type A ou B.

Un autre objet de l'invention concerne l'utilisation d'au moins un polypeptide de l'invention dans une composition ou pour la préparation d'une composition, le polypeptide ou la composition étant destinés à traiter les dysfonctionnements de la prolifération ou de la différenciation épidermique, les peaux sèches, l'hyperkératose, la parakératose, le psoriasis, les ichtyoses et/ou les néoplasies.
Elle concerne aussi l'utilisation d'au moins un polypeptide de l'invention comme agent pour traiter les signes du vieillissement cutané et/ou lutter contre les effets du rayonnement ultra-violet, particulièrement de type A ou B.

Un autre objet de l'invention est encore un procédé de traitement cosmétique destiné à lutter contre les signes cutanés des dysfonctionnements de la prolifération et/ou de la différenciation épidermique comme les peaux sèches, l'hyperkératose, la parakératose, le psoriasis, les ichtyoses et/ou les néoplasies, les signes du vieillissement cutané et/ou les effets du rayonnement ultra-violet, particulièrement de type A ou B, caractérisé par le fait que l'on applique sur la peau, les muqueuses et/ou les fibres kératiniques une composition cosmétique comprenant au moins un polypeptide de l'invention.

Le procédé de traitement de l'invention est un procédé cosmétique destiné à améliorer l'aspect esthétique de l'individu subissant des troubles de la prolifération et/ou de la différenciation épidermique.

La quantité de polypeptide contenue dans la composition de l'invention est, bien entendu, fonction de l'effet recherché et peut donc varier dans une large mesure.

Pour donner un ordre de grandeur, la composition peut contenir le polypeptide de l'invention en une quantité représentant de 0,00001% à 50% du poids total de la composition et préférentiellement en une quantité représentant de 0,001% à 10% du poids total de la composition et encore plus préférentiellement en une quantité représentant de 0,1% à 1% du poids total de la composition.

L'invention a également pour objet l'utilisation du polypeptide de l'invention ou de ses fragments de protéolyse et de tout peptide synthétique déduit de sa séquence, pour préparer ou purifier, éventuellement à partir d'épiderme, toute molécule, structurale ou fonctionnelle, susceptible de se lier spécifiquement audit polypeptide isolé ou auxdits fragments de protéolyse isolés ou audit peptide synthétique.

A cet égard, la demanderesse a démontré que le polypeptide de l'invention peut par exemple se lier à au moins une cible protéique choisie parmi les transglutaminases, particulièrement la transglutaminase 3, la galectine 7, les annexines, particulièrement l'annexines 2, la MRP14 ou calgranuline B, les heparanases, la HSP 27, la SCCE ou encore la protéine L9 ribosomale de 60S.
Dans la mesure où l'on sait que les transglutaminases jouent un rôle important dans la formation des enveloppes cornées, on peut supposer que la CLSP joue un rôle régulateur de la formation de senveloppes cormées au travers de la fixation à la tranglutaminase.

Ainsi l'invention a pour objet l'utilisation du polypeptide de l'invention ou de ses fragments de protéolyse et de tout peptide synthétique déduit de sa séquence pour réguler les tranglutaminases; particutièrement la transglutaminase 3 et ainsi réguler la formation de la couche cornée de l'épiderme.

Dans la mesure où le polypeptide de l'invention appartient à la famille de Calmodulines et que l'on sait que les polypeptides de cette famille sont en général des second-messagers répondant à un stimulus (le taux de calcium intracellulaire) via une fixation à leur protéine cible, il est possible d'utiliser le polypeptide de l'invention pour préparer ou purifier, éventuellement à partir d'épiderme, toute molécule, structurale ou fonctionnelle, susceptible de moduler l'interaction entre le polypeptide de l'invention et ses éventuels ligands.

Ainsi, l'invention la pour objet l'utilisation du polypeptide de l'invention ou de ses fragments de protéolyse et de tout peptide synthétique déduit de sa séquence, pour préparer ou purifier, éventuellement à partir d'épiderme, toute molécule, structurale ou fonctionnelle, susceptible de moduler l'interaction entre le polypeptide de l'invention et ses éventuels ligands.

L'invention a également pour objet l'utilisation du polypeptide de l'invention ou de ses fragments de protéolyse et de tout peptide synthétique déduit de sa séquence, pour préparer des antisérums et/ou des anticorps monoclonaux spécfiques, visant notamment à purifier cette protéine et ses fragments. Par extension, l'invention a également pour objet toute utilisation de ladite séquence pour produire des anticorps ou fragments d'anticorps recombinants, quel que soit le système biologique utilisé pour produire ces derniers.

L'invention a encore pour objet un anticorps poly- ou monoclonal caractérisé par le fait qu'il reconnaît spécifiquement le polypeptide de l'invention.

L'anticorps peut être un anticorps prépare par immunisation de toute-espèce animale utilisable à cette fin, particulièrement le lapin. L'anticorps peut être préparé par immunisation à l'aide du polypeptide de l'invention que celui-ci soit d'origine naturelle ou synthétique, purifié ou non.

On sait qu'une protéine est synthétisée dans les cellules à partir d'une matrice d'acides désoxyribonucléiques codant pour ladite protéine. On sait également que le code génétique est dégénéré. Ainsi, la séquence d'acides aminés du polypeptide de l'invention peut être issue de différentes séquences d'acides désoxyribonucléiques, naturelles ou synthétiques. Par séquence d'acides désoxyribonucléiques synthétique, on entend ici toute séquence obtenue chimiquement ou par manipulation génétique.

Lesdites séquences d'acides désoxyribonucléiques peuvent être issues de toutes origines possibles à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

La demanderesse a, isolé, purifié et séquence par des techniques de biologie moléculaire, en particulier le criblage de banques d'expression d'acides désoxyribonucléiques complémentaires préparées à partir d'épiderme humain, un fragment d'acides désoxyribonucléiques codant pour le polypeptide de l'invention.

L'invention a donc également pour objet une composition cosmétique ou pharmaceutique comprenant, dans un milieu physiologiquement acceptable, toutes séquences d'acides désoxyribonucléiques, naturelles ou synthétiques, dont tout ou partie code pour la séquence primaire d'acides aminés du polypeptide de l'invention.

Au cours de ces travaux, la demanderesse a pu isoler et purifier une séquence d'acides désoxyribonucléiques codant pour la séquence primaire d'acides aminés du polypeptide de l'invention à partir de peau humaine.

L'invention a pour objet un fragment d'acide désoxyribonucléique isolé et purifié correspondant à la séquence nucléotidique codante SEQ ID NO : 2 suivante :

L'invention a aussi objet tout fragment d'acide désoxyribonucléique isolé et purifié comprenant au moins la séquence nucléotidique codante SÉQ ID NO : 2.

L'invention a également pour objet tout polynucléotide, acide ribonucléique ou désoxynbonucléique, sens ou antisens, correspondants au moins à la séquence nucléotidique codante SEQ ID NO : 2.

Le fragment d'ADN correspondant à la séquence SEQ ID NO : 2 peut être introduit dans un vecteur d'expression permettant ainsi la synthèse d'une protéine dite recombinante correspondant à la CLSP.
Par protéine recombinante on entend ici tout élément peptidique correspondant à tout ou partie de la séquence peptidique de la CLSP, obtenu de manière artificielle après introduction et expression de tout ou partie de l'ADN complémentaire de la CLSP dans un quelconque vecteur d'expression, quel que soit l'organisme qui permet cette expression.

Particulièrement selon l'invention, la CLSP ou une partie de la CLSP, peut être produite après introduction de tout ou partie de la séquence nucléotidique codante SEQ ID NO : 2 dans un vecteur d'expression comme par exemple le vecteur pGex-2T (Amersham Pharmacia Biotech Inc.) et expression dans E. Coli.

L'invention a donc également pour objet un vecteur d'expression recombinant contenant tout ou partie de la séquence nucléotidique codante SEQ ID NO : 2.

L'invention a encore pour objet une protéine recombinante correspondant à tout ou partie de la séquence SEQ ID NO : 1, particulièrement celle obtenue par expression d'un vecteur d'expression pGex-2T contenant tout ou partie de la séquence codante SEQ ID NO : 2.

L'invention a également pour objet une composition cosmétique ou pharmaceutique comprenant, dans un milieu physiologiquement acceptable, au moins un fragment d'acide désoxyribonucléique isolé et purifié comprenant au moins la séquence nucléotidique codante SEQ ID NO : 2

L'invention a également pour objet une composition cosmétique ou pharmaceutique comprenant, dans un milieu physiologiquement acceptable, une séquence d'acides ribonucléiques sens ou antisens correspondants à ladite séquence SEQ ID NO : 2.

Elle a également pour objet l'utilisation desdites séquences d'acides désoxyribonucléiques pour la production du polypeptide de l'invention ou d'un acide ribonucléique correspondant par toute technique connue comme par exemple la synthèse in vitro, à partir de milieux reconstitués ou la synthèse par des organismes ou microorganismes.

Quelle que soit leur nature, les compositions de l'invention peuvent être ingérées, injectées ou appliquées sur la peau (sur toute zone cutanée du corps) ou les muqueuses (buccale, jugale, gingivale, génitale, conjonctivale, ...).

Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (HIE) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses ou encore sous forme de compositions pour aérosol comprenant également un agent propulseur sous pression. Ces compositions sont préparées selon les méthodes usuelles.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquiltage, des laits corporels de protection ou de soin, des laits anti-solaires, des laits après-soleil, des lotions, gels ou mousses pour le soin de la peau, comme des lotions de nettoyage, des lotions anti-solaires, des lotions après-soleil, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères, l'ichthyose.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour les soins du cuir chevelu, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, les compositions antipelliculaires, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition.
Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.
Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéines, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins une Iridacée à d'autres agents actifs destinés notamment à la prévention et/ou au traitement des affections cutanées. Parmi ces agents actifs, on peut citer à titre d'exemple:
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone :

- les antibactériens tels que le phosphate de clindamycine, l'érythromycine ou les antibiotiques de la classe des tétracyclines ;
- les antiparasitaires, en particulier le métronidazole, te crotamiton ou les pyréthrinoïdes ;
- les antifongiques, en particulier les composés appartenant à la classe des imidazoles tels que l'éconazole, le kétoconazote ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens; tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β- hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents hydratants comme le glycérol et ses dérivés ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

Ainsi, selon un mode particulier, la composition selon l'invention comprend également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents diminuant la différenciation et/ou la prolifération et/ou la pigmentation cutanée.

### Exemple 1

Matériels et méthodes :
La bibliothèque d'ADNc de kératinocytes clonée dans λgt11 (origine des ARNm = kératinocytes de prépuces humains adultes) provient de Clontech (Palo Alto, CA, USA).

Les oligomères utilisés dans les différentes expériences sont présentés ci-après

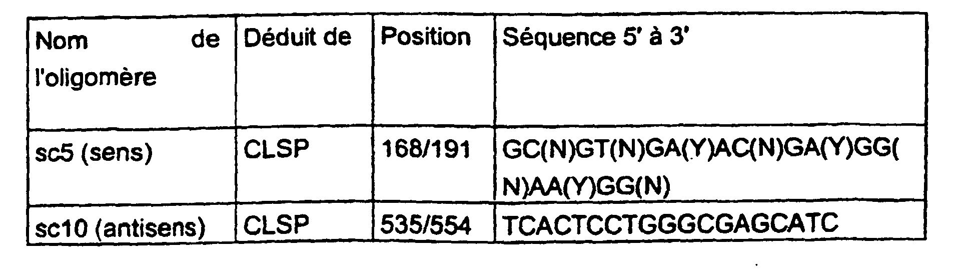

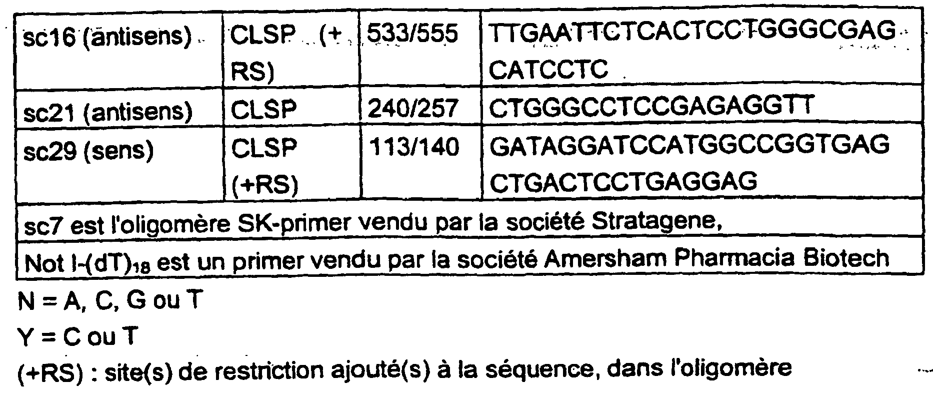

L'oligomère dégénéré sc5 est basé sur la séquence d'acides aminés SEQ ID NO:1. Tous les autres oligomères déduits de la CLSP et leur position sont déduits de la séquence nucléotidique SEQ ID NO:2.
Not I-(dT)₁₈ est conçu pour se fixer au longues séries de A, par exemple les polya+. Utilisés dans les réactions de transcription réverse, Not I-(dT)₁₈ se fixe au polyA+.

### 1. Préparations à partir de stratum comeum humain

Tampons :
I : SDS 0,3% ; tris-HCl 28 mM ; tris-base 22mM
2D : 8M urée ; 2% CHAPS (3-(3-cholamidopropyl)diméthylamonio-1-propane-sulfonate) ; Dithiothréitol 20 mM ; 0,5% Imobilin pH gradient Buffer (Amersham Pharmacia Biotech), pH=3 ;

Pour la purification de la CLSP, des extraits acétoniques de stratum comeum ont été préparés selon la méthode décrite par Lundstrom et Egelrüd dans Acta Derm. Venerol., 1991, 71 : 471-474.
78 mg de poudre acétoniques ont ainsi été préparés.
2,5ml de tampon 1 sont ajoutés aux 78 mg de poudre acétonique et le tout est pottérisé, porté à ébullition pendant 10 minutes puis repotterisé. La solution est alors centrifugée à 10000 g pendant 10 minutes. Le surnageant est recueilli et filtré à 0,22 M. On obtient ainsi 2 ml de surnageant SI.
On ajoute alors au surnageant SI 10 ml d'acétone froide (10V/2V). On laisse 10 minutes au froid (glace pilée) et ion centrifuge à 9400 g pendant 20 minutes à 4°C..
Le surnageant est alors éliminé et le culot séché à température ambiante pendant 20 minutes Le culot est alors repris dans 300 l de tampon 2D. On obtient ainsi l'extrait El.

### 2. Gel bidimensionnel

La séparation en 2 dimensions des protéines contenues dans l'extrait El est réalisée sur un appareil de marque Pharmacia (modèle multiphor) selon les recommandations du fournisseur.
La coloration des spots, la récupération de ceux-ci et le séquençage des polypeptides qu'ils contenaient ont été réalisés selon les techniques standards décrites dans "gel electrophoresis of proteins" (IRL press), ou encore "a practical guide to protein and peptide puriifcation for microsequencing, (Paul Matsudaira éditeur, seconde édition 1993).

### 3 Clonage et séquençage de l'ADNc CLSP :

Les ARN totaux de kératinocytes d'un équivalent d'épiderme (Episkin) ont été préparés à l'aide d'un kit de préparation RNeasy Kit et purifiés à l'aide d'un kit QIAshredder column de la marque Qiagen selon les prescriptions du fournisseur.
Les ADN complémentaires des ARN ainsi préparés ont été synthétisés à l'aide d'un kit First strand cDNA Synthesis kit de marque Amersham Pharmacia Biotech selon les prescriptions du fournisseur en utilisant l'oligomère Notl-d(T)18.
Un ADNc de 775 paires de bases ainsi obtenu a été amplifié par des réactions de polymérisation en chaîne (PCR) dans un appareil à cycles thermiques de marque Perkin-Elmer (modèle Thermocycler) en utilisant une polymérase pfu de marque Stratagene et le couple d'oligomère sc5/Notl-d(T)18. Un séquençage de ce fragment a permis d'en déterminer l'enchaînement en nucléotides.
Afin d'obtenir la séquence complète de l'ADNc de la CLSP, des réactions de PCR ont été réalisées à l'aide des couples d'oligomères suivants sc10/sc7, sc7/sc21, sc16/sc29 dans des conditions d'expérience standards, sur une banque d'ADN complémentaires de kératinocytes clonés dans le phage gt11 (Stratagene).

Les recherches d'homologies sont effectuées via le programme WU-Blast sur le site Internet Expasy Swissprot, la détection d'éventuels sites de clivage est effectuée via le programme Omiga 1.1 PC (Oxford Molecular, Oxford, U.K.).

Construction et expression d'une protéine CLSP recombinante :
L'ADNc de la CLSP est introduit dans le vecteur pGex-2T (Pharmacia Biotech Inc.) par coupure/ligature aux sites de restriction BamH-1/EcoRI. La construction pour laquelle la séquence codante de la CLSP est en phase avec la séquence de la glutathione S-transferase contenue dans le vecteur, est alors introduite dans la bactérie E. Coli BL21. La protéine recombinante exprimée par les bactéries peut être coupée par la thrombine, la construction étant telle que la protéine de fusion obtenue porte un site de coupure par cette protéase. L'ensemble de ces expériences a été réalisé en appliquant strictement les différents protocoles des fournisseurs.

Fixation du calcium par la CLSP :
La protéine de fusion obtenue ci-dessus est déposée sur un gel de type SDS-PAGE en présence ou en absence d'éthylène diamine tétraacétate (EDTA), agent chélateur des ions calcium, selon le protocole décrit par Burgess et col. (BBA, 623 (1980), 257-270).

Chromatographie d'affinité :
Des colonne de chromatographie d'affinité ont été réalisée avec 1ml de bille.
Le polypeptide de l'invention a été immobilisé sur des billes de sépharose activées au bromure de cyanogène provenant de chez Amersham Pharmacia Biotech et selon les recommandations du fabriquant en utilisant 1,3 mg de polypeptide par millilitre de billes.
L'efficacité de couplage a été de 0,95 mg de polypeptide par millilitre de billes.
De l'épiderme humain (0,83g de peau d'abdomen provenant de plasties chirurgicales et 100g de stratum corneum plantaire) ont été homogénéisés au polytron dans 10 ml et 120 ml respectivement de tampon Hepes 10 mM, pH 7,4, contenant 150 mM de chlorure_de sodium, 0,1%(poids/volume) de triton X100 et un mélange d'inhibiteurs de protéases (Complete™ EDTA-free de Roche Molecular additionné de 1 M depepstatin).
La suspension est alors centrifugée à 10000 g pendant 10 minutes à 4°C.. Le surnageant est filtré à 0,22 et ajusté à 2,5 mM de chlorure de calcium avant passage à température ambiante sur la colonne d'affinité. Les colonnes sont alors lavées avec 10 volumes de tampon Hepes 10 mM, pH 7,4, contenant 150 mM de chlorure de sodium, 0,1% (poids/volume) de triton X100 et un mélange d'inhibiteurs de protéases. L'élution est effectuées en utilisant le même tampon contenant 5 mM-d'EDTA au lieu de 2, mM de chlorure de calcium.
Les protéines éluées sont soumises à une électrophorèse en gel dénaturant (SDS-PAGE) et après migration, colorées à l'argent à l'aide du kit de coloration à l'argent de la société Amersham Pharmacia Biotech et selon les recommandations du fabriquant.

### RESULTATS:

Purification et caractérisation de la CLSP
Parmi tous les spots analysés à partir du gel bidimensionnel, la séquence déduite d'un des spots a donné une homologie de séquence avec la Calmoduline suffisamment importante pour qu'il soit retenu mais suffisamment différente pour que son analyse soit poursuivie.
A partir des éléments de séquence en acides aminés obtenus et par les techniques de biologies moléculaires ci-dessus décrites, il a été possible d'isoler et de purifier la séquence SEQ ID NO : 1.
Les homologies de séquence entre cette protéine et la Calmoduline laissent à penser que cette protéine doit avoir des propriétés similaires de celles de la Calmoduline.
Quatre sites de liaison au calcium peuvent être mis en évidence dans cette séquence, à savoir les sites compris entre les acides aminés 21 et 32, 57 et 68, 91 et 102, et 127 et 138.
Les expériences de migration électrophorétique, particulièrement avec la protéine recombinante, conduites en présence ou en absence d'ions calcium confirment que la protéine fixe le calcium.

Cette séquence correspond donc à une protéine complète, qui fixe le calcium, qui n'est pas la Calmoduline et qui est présente dans le stratum comeum de l'épiderme humain.

Clonage de l'ADN de la SCTE :
Par les techniques ci-dessus décrites, un ADN de 858 paires de bases, a été isolé. Ce fragment d'ADN comporte la séquence codante intégrale de la CLSP, du codon ATG en position 114 jusqu'au codon stop en position 552.

Chromatographie d'affinité :
Le séquençage de l'une des protéines retenue sur la colonne d'affinité réalisée avec le polypeptide de l'invention a révélé qu'il s'agit d'une transglutaminase et particulièrement de la transglutaminase 3.
D'autres expériences ont révélé que la galectine 7, les annexines, particulièrement l'annexines 2, la MRP14 ou calgranuline B, les heparanases particulièrement l'heparanase III, la Heat Shock P 27, la SCCE ou encore la protéine L9 ribosomale de 60S se lient également au polypeptide de l'invention.

## Revendications

1. Polypeptide naturel ou synthétique purifié, **caractérisé par le fait qu'**il comprend la séquence d'acides aminés SEQ ID NO : 1 :

2. Polypeptide selon la revendication 1, **caractérisé par le fait qu'**il est purifié à partir de peau humaine.

3. Polypeptide selon l'une quelconque des revendications précédentes, **caractérisé par le fait qu'**il est purifié à partir d'épiderme humain.

4. Polypeptide naturel ou synthétique dont la séquence répond à la séquence d'acides aminés SEQ ID NO 1 selon la revendication 1.

5. Polypeptide selon l'une quelconque des revendications précédentes, **caractérisé par le fait qu'**il a un point isoélectrique théorique de 4.075.

6. Polypeptide selon l'une quelconque des revendications précédentes, **caractérisé par le fait qu'**il a un poids moléculaire théorique de 15,9 kilodaltons (kD).

7. Polypeptide selon l'une quelconque des revendications précédentes, **caractérisé par le fait qu'**il présente dans sa séquence primaire au moins un site de fixation du calcium.

8. Polypeptide selon l'une quelconque des revendications précédentes, **caractérisé par le fait qu'**il fixe le calcium.

9. Composition **caractérisée par le fait qu'**elle comprend dans un milieu physiologiquement acceptable, au moins un polypeptide tel que défini dans l'une quelconque des revendications 1 à 8.

10. Composition selon la revendication 9, **caractérisée en ce qu'**elle est cosmétique ou pharmaceutique.

11. Utilisation d'au moins un polypeptide tel que défini dans l'une quelconque des revendications 1 à 8 pour préparer une composition destinée à traiter les dysfonctionnements de la prolifération ou de la différenciation épidermique normales ou pathologiques.

12. Utilisation d'au moins un polypeptide tel que défini dans l'une quelconque des revendications 1 à 8, pour préparer une composition destinée à traiter les peaux sèches, l'hyperkéralose, la parakératose, le psoriasis, les ichtyoses, les néoplasies.

13. Utilisation d'au moins un polypeptide selon l'une des revendications 11 ou 12, **caractérisée par le fait que** la composition est destinée à une application cosmétique ou pharmaceutique.

14. Utilisation d'au moins un polypeptide tel que défini dans l'une quelconque des revendications 1 à 8. pour préparer une composition destinée à réguler la transglutaminase 3.

15. Utilisation d'au moins un polypeptide tel que défini dans l'une quelconque des revendications 1 à 8. pour préparer une composition destinée à réguler la formation de la couche cornée de l'épiderme.

16. Utilisation d'au moins un polypeptide pour préparer une composition selon l'une quelconque des revendications 9 à 15, **caractérisée en ce que** ladite composition est une composition cosmétique pour application topique.

17. Fragment d'acide désoxyribonucléique isolé codant pour le polypeptide tel que défini dans l'une des revendications 1 à 8.

18. Fragment d'acide désoxyribonucléique isolé comprenant au moins la séquence nucléotidique codante SEQ ID NO : 2 suivante :

19. Composition cosmétique ou pharmaceutique, **caractérisée par le fait qu'**elle comprend, dans un milieu physiologiquement acceptable au moins une séquence nucléotidique telle que décrite dans la revendication 18.

20. Utilisation d'au moins une séquence d'acide désoxyribonucléique telle que décrite dans la revendication 18 pour préparer un polypeptide ou un mélange de polypeptides.

21. Vecteur d'expression recombinant contenant la séquence nucléotidique codante SEQ ID NO: 2.

22. Composition cosmétique ou pharmaceutique **caractérisé en ce qu'**elle comprend, dans un milieu physiologiquement acceptable, une protéine recombinante correspondant à la séquence SEQ ID NO : 1.

23. Utilisation d'au moins une séquence d'acide désoxyribonucléique telle que décrite dans la revendication 18 pour préparer un acide ribonucléique.

24. Utilisation d'au moins un polypeptide isolé ou de tout peptide synthétique tel que décrit dans les revendications 1 à 8. pour préparer ou purifier toute molécule susceptible de se lier spécifiquement audit polypeptide purifié ou audit peptide synthétique.

25. Utilisation d'au moins un polypeptide isolé ou de tout peptide synthétique tel que décrit dans les revendications 1 à 8, pour préparer ou purifier des antisérums ou des anticorps monoclonaux spécifiques.

26. Anticorps poly- ou monoclonal **caractérisé par le fait qu'**il reconnaît Spécifiquement le polypeptide tel que décrit dans les revendications 1 à 8.

## Claims

1. Purified, natural or synthetic polypeptide, **characterized in that** it comprises the amino acid sequence SEQ ID NO: 1:

2. Polypeptide according to Claim 1, **characterized in that** it is purified from human skin.

3. Polypeptide according to either one of the preceding claims, **characterized in that** it is purified from human epidermis.

4. Natural or synthetic polypeptide whose sequence corresponds to the amino acid sequence SEQ ID NO. 1 according to Claim 1.

5. Polypeptide according to any one of the preceding claims, **characterized in that** it has a theoretical isoelectric point of 4.075.

6. Polypeptide according to any one of the preceding claims, **characterized in that** it has a theoretical molecular weight of 15.9 kilodaltons (kD).

7. Polypeptide according to any one of the preceding claims, **characterized in that** it has, in its primary sequence, at least one calcium-binding site.

8. Polypeptide according to any one of the preceding claims, **characterized in that** it binds calcium.

9. Composition, **characterized in that** it comprises, in a physiologically acceptable medium, at least one polypeptide as defined in any one of Claims 1 to 8.

10. Composition according to Claim 9, **characterized in that** it is cosmetic or pharmaceutical.

11. Use of at least one polypeptide as defined in any one of Claims 1 to 8, for preparing a composition for use in the treatment of normal or pathological epidermal proliferation or differentiation dysfunctions.

12. Use of at least one polypeptide as defined in any one of Claims 1 to 8, for preparing a composition for use in the treatment of dry skin, hyperkeratosis, parakeratosis, psoriasis, ichthyosis and neoplasms.

13. Use of at least one polypeptide according to either of Claims 11 and 12, **characterized in that** the composition is intended for cosmetic or pharmaceutical application.

14. Use of at least one polypeptide as defined in any one of Claims 1 to 8, for preparing a composition for use in the regulation of transglutaminase 3.

15. Use of at least one polypeptide as defined in any one of Claims 1 to 8, for preparing a composition for use in regulating the formation of the cornefied layer of the epidermis.

16. Use of at least one polypeptide for preparing a composition according to any one of Claims 9 to 15, **characterized in that** said composition is a cosmetic composition for topical application.

17. Isolated deoxyribonucleic acid fragment encoding the polypeptide as defined in one of Claims 1 to 8.

18. Isolated deoxyribonucleic acid fragment comprising at least the coding nucleotide sequence SEQ ID NO: 2 below:

19. Cosmetic or pharmaceutical composition, **characterized in that** it comprises, in a physiologically acceptable medium, at least one nucleotide sequence as described in Claim 18.

20. Use of at least one deoxyribonucleic acid sequence as described in Claim 18, for preparing a polypeptide or a mixture of polypeptides.

21. Recombinant expression vector containing the coding nucleotide sequence SEQ ID NO: 2.

22. Cosmetic or pharmaceutical composition, **characterized in that** it comprises, in a physiologically acceptable medium, a recombinant protein corresponding to the sequence SEQ ID NO: 1.

23. Use of at least one deoxyribonucleic acid sequence as described in Claim 18, for preparing a ribonucleic acid.

24. Use of at least one isolated polypeptide or of any synthetic peptide as described in Claims 1 to 8, for preparing or purifying any molecule capable of binding specifically to said purified polypeptide or to said synthetic peptide.

25. Use of at least one isolated polypeptide or of any synthetic peptide as described in Claims 1 to 8, for preparing or purifying specific monoclonal antibodies or antisera.

26. Polyclonal or monoclonal antibody, **characterized in that** it specifically recognizes the polypeptide as described in Claims 1 to 8.

## Patentansprüche

1. Gereinigtes natürliches oder synthetisches Polypeptid, **dadurch gekennzeichnet, dass** es die Aminosäuresequenz des Sequenzprotokolls SEQ ID NO : 1 aufweist:

2. Polypeptid nach Anspruch 1, **dadurch gekennzeichnet, dass** es durch Reinigung ausgehend von menschlicher Haut erhalten ist.

3. Polypeptid nach einem der vorhergehenden Ansprüche, **dadurch gekennzeichnet, dass** es durch Reinigung ausgehend von menschlicher Epidermis erhalten ist.

4. Natürliches oder synthetisches Polypeptid, dessen Sequenz der Aminosäuresequenz des Sequenzprotokolls SEQ ID NO : 1 nach Anspruch 1 entspricht.

5. Polypeptid nach einem der vorhergehenden Ansprüche, **dadurch gekennzeichnet, dass** es einen theoretischen isoelektrischen Punkt von 4,075 besitzt.

6. Polypeptid nach einem der vorhergehenden Ansprüche, **dadurch gekennzeichnet, dass** es ein theoretisches Molekulargewicht von 15,9 Kilodalton (kD) aufweist.

7. Polypeptid nach einem der vorhergehenden Ansprüche, **dadurch gekennzeichnet, dass** es in seiner primären Sequenz mindestens eine Bindungsstelle für Calcium aufweist.

8. Polypeptid nach einem der vorhergehenden Ansprüche, **dadurch gekennzeichnet, dass** es Calcium bindet.

9. Zusammensetzung, **dadurch gekennzeichnet, dass** sie in einem physiologisch akzeptablen Medium mindestens ein Polypeptid enthält, wie es in einem der Ansprüche 1 bis 8 definiert ist.

10. Zusammensetzung nach Anspruch 9, **dadurch gekennzeichnet, dass** es sich um eine kosmetische oder pharmazeutische Zusammensetzung handelt.

11. Verwendung mindestens eines Polypeptids wie in einem der Ansprüche 1 bis 8 definiert zur Herstellung einer Zusammensetzung, die zur Behandlung von normalen oder pathologischen Dysfunktionen der Proliferation oder der Differenzierung der Epidermis bestimmt ist.

12. Verwendung mindestens eines Polypeptids wie in einem der Ansprüche 1 bis 8 definiert zur Herstellung einer Zusammensetzung, die zur Behandlung von trockener Haut, Hyperkeratose, Parakeratose, Psoriasis, Ichthyosen und Neoplasien bestimmt ist.

13. Verwendung mindestens eines Polypeptids nach einem der Ansprüche 11 oder 12, **dadurch gekennzeichnet, dass** die Zusammensetzung zur kosmetischen oder pharmazeutischen Anwendung bestimmt ist.

14. Verwendung mindestens eines Polypeptids wie in einem der Ansprüche 1 bis 8 definiert zur Herstellung einer Zusammensetzung, die zur Regulierung der Transglutaminase 3 vorgesehen ist.

15. Verwendung mindestens eines Polypeptids wie in einem der Ansprüche 1 bis 8 definiert zur Herstellung einer Zusammensetzung, die zur Regulierung der Bildung der Hornschicht der Epidermis vorgesehen ist.

16. Verwendung mindestens eines Polypeptids zur Herstellung einer Zusammensetzung nach einem der Ansprüche 9 bis 15,
**dadurch gekennzeichnet, dass** die Zusammensetzung eine kosmetische Zusammensetzung zur topischen Anwendung ist.

17. Isoliertes Desoxyribonucleinsäure-Fragment, das ein Polypeptid codiert, das wie in einem der Ansprüche 1 bis 8 definiert ist.

18. Isoliertes Desoxyribonukleinsäure-Fragment, das mindestens die codierende Nucleotidsequenz des nachstehenden Sequenzprotokolls SEQ ID NO : 2 aufweist:

19. Kosmetische oder pharmazeutische Zusammensetzung, **dadurch gekennzeichnet, dass** sie in einem physiologisch akzeptablen Medium mindestens eine Nucleotidsequenz enthält, wie sie in Anspruch 18 beschrieben ist.

20. Verwendung mindestens einer Desoxyribonucleinsäure-Sequenz wie in Anspruch 18 beschrieben zur Herstellung eines Polypeptids oder eines Gemisches von Polypeptiden.

21. Rekombinanter Expressionsvektor, der die codierende Nucleotidsequenz des Sequenzprotokolls SEQ ID NO : 2 enthält.

22. Kosmetische oder pharmazeutische Zusammensetzung, **dadurch gekennzeichnet, dass** sie in einem physiologisch akzeptablen Medium ein rekombinantes Protein enthält, das der Sequenz des Sequenzprotokolls SEQ ID NO : 1 entspricht.

23. Verwendung mindestens einer Desoxyribonucleinsäure-Sequenz wie in Anspruch 18 beschrieben zur Herstellung einer Ribonucleinsäure.

24. Verwendung mindestens eines isolierten Polypeptids oder eines beliebigen synthetischen Peptids wie in den Ansprüchen 1 bis 8 beschrieben zur Herstellung oder Reinigung von Molekülen, die in der Lage sind, eine spezifische Bindung mit dem gereinigten Polypeptid oder dem synthetischen Peptid einzugehen.

25. Verwendung mindestens eines isolierten Polypeptids oder eines beliebigen synthetischen Peptids wie in den Ansprüchen 1 bis 8 beschrieben zur Herstellung oder Reinigung von Antiseren oder spezifischen monoklonalen Antikörpern.

26. Polyklonaler oder monoklonaler Antikörper, **dadurch gekennzeichnet, dass** er das in den Ansprüchen 1 bis 8 beschriebene Polypeptid spezifisch erkennt.
